(19) Europäisches Patentamt
European Patent Office
Office européen des brevets

(11) EP 4 772 271 A1

(12) # EUROPEAN PATENT APPLICATION
published in accordance with Art. 153(4) EPC

(43) Date of publication:
**08.07.2026 Bulletin 2026/28**

(21) Application number: **24859855.9**

(22) Date of filing: **28.08.2024**

(51) International Patent Classification (IPC):
***B01J 20/281*** *(2006.01)* ***B01D 15/38*** *(2006.01)*
***B01J 20/28*** *(2006.01)* ***B01J 20/285*** *(2006.01)*
***C07K 1/16*** *(2006.01)* ***C07K 16/00*** *(2006.01)*
***G01N 30/88*** *(2006.01)*

(52) Cooperative Patent Classification (CPC):
**B01D 15/38; B01J 20/28; B01J 20/281; B01J 20/285; C07K 1/16; C07K 16/00; G01N 30/88**

(86) International application number:
**PCT/JP2024/030807**

(87) International publication number:
**WO 2025/047826 (06.03.2025 Gazette 2025/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.08.2023 JP 2023141353**

(71) Applicant: **JNC Corporation**
**Chiyoda-ku**
**Tokyo 100-8105 (JP)**

(72) Inventors:
- **MORI, Chigusa**
  **Yokohama-shi, Kanagawa 236-8605 (JP)**
- **IWAMOTO, Eri**
  **Yokohama-shi, Kanagawa 236-8605 (JP)**

(74) Representative: **Becker, Eberhard**
**Becker Kurig & Partner**
**Patentanwälte mbB**
**Bavariastraße 7**
**80336 München (DE)**

(54) **CHROMATOGRAPHY CARRIER AND METHOD FOR PURIFYING ANTIBODY**

(57) One embodiment of the present invention provides a chromatography carrier which includes a base carrier that includes porous particles, and a ligand that is immobilized on the base carrier. The ligand is a polyallylamine represented by formula (1), or a polyallylamine, in which some amino groups are modified, or a salt thereof. (1): -NH-Z-X (In formula (1), Z represents a divalent hydrophobic group, and X represents $NH_2$ or a heterocyclic group that contains a nitrogen atom.) The ligand is immobilized on the base carrier via one or more kinds of active groups that are reactive to the ligand.

EP 4 772 271 A1

## Description

### Technical Field

The present invention relates to a chromatography carrier and a method for purifying an antibody using the same.

### Background Art

It is widely known to purify biopharmaceuticals using chromatography technology, and target substances and impurities are separated by utilizing various intermolecular interactions. Such examples include ion exchange chromatography utilizing electrostatic interactions, hydrophobic interaction chromatography utilizing hydrophobic interactions, and protein A affinity chromatography utilizing affinity interactions with antibodies.

Ion exchange chromatography is the most commonly used technique in the purification of biopharmaceuticals. In ion exchange chromatography, for samples with high electrical conductivity in the processing solution, it is necessary to reduce the electrical conductivity to, for example, 5 mS/cm by dilution or desalting before performing adsorption treatment.

Hydrophobic interaction chromatography is the second most commonly used technique after ion exchange chromatography. In hydrophobic interaction chromatography, in order to adsorb proteins and the like, it is necessary to include a high concentration of ammonium sulfate or sodium sulfate at, for example, about 2.0 mol/liter in the adsorption buffer solution. Consequently, processing a large amount of culture solution requires a large amount of salt, which becomes a factor of higher manufacturing costs.

To compensate for such drawbacks of ion exchange chromatography and hydrophobic interaction chromatography, significant efforts have been directed toward the development of multimodal chromatography carriers in recent years, which introduces two or more types of ligands capable of interacting based on different principles into the same base carrier.

Multimodal chromatography carriers exhibit different selectivity from chromatography carriers that carry ligands having only ion exchange groups or only hydrophobic groups.

For example, a method for purifying antibodies using a carrier having anion exchange groups and aromatic groups in a flow-through mode is known (Patent Document 1). Further, a method for purifying antibodies using a carrier in which some primary amino groups are modified with hydrophobic groups is known (Patent Document 2).

Furthermore, a method for purifying biomolecules using a chromatography carrier in which ligands including anion exchange groups, alkyl groups, and aryl groups or heteroaryl groups are bound to a base carrier via spacers is also known (Patent Document 3). In particular, it has been found that chromatography carriers having amino groups and hydrophobic groups adsorb proteins under conditions of high conductivity (Non-Patent Document 1).

However, the conventional multimodal chromatography carriers may sometimes lack sufficient separation characteristics between target proteins (for example, antibodies) and impurity proteins (for example, host cell protein (HCP)), causing the problem that the target proteins are adsorbed along with impurity proteins. As a result, it has been difficult to obtain target proteins with a high recovery rate.

### Related Art Documents

#### Patent Documents

Patent Document 1: Japanese Patent No. 5064225
Patent Document 2: International Publication No. 2018/092691
Patent Document 3: International Publication No. 2021/046284

#### Non-Patent Documents

Non-Patent Document 1: Journal of Chromatography A, 1016 (2003), 21-33

### SUMMARY OF INVENTION

#### Problem to be Solved by the Invention

The present invention aims to provide a chromatography carrier that is capable of purifying a target substance with a high recovery rate, and a method for purifying an antibody using the same.

Means for Solving the Problem

Based on the above-mentioned problem, the present inventors conducted intensive research and found that using a chromatography carrier formed by immobilizing a ligand having a specific structure including hydrophobic groups and amino groups to a base carrier makes it possible to purify a target substance with a high recovery rate, leading to completion of the present invention.

The present invention is, for example, as follows.

[1] A chromatography carrier, including:

a base carrier including porous particles; and a ligand immobilized on the base carrier,
wherein the ligand is represented by the following formula (1), or is a polyallylamine having some amino groups modified or a salt thereof,

-NH-Z-X (1)

(in formula (1), Z represents a divalent hydrophobic group; X represents $NH_2$ or represents a heterocyclic group including a nitrogen atom)
the polyallylamine having some amino groups modified or a salt thereof includes a structural unit represented by the following formula (2) and a structural unit represented by the following formula (3) or formula (3a), and has a weight average molecular weight of 1000 to 500000,

[Chemical Formula 1]

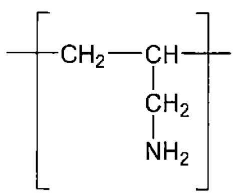
(2)

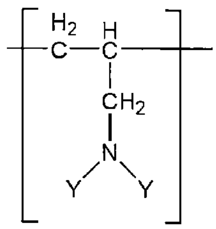
(3)

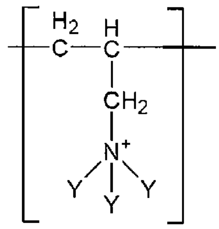
(3a)

[in formulas (3) and (3a), Y is each independently selected from the group consisting of a hydrogen atom, an alkyl group, an aryl group, $-COR^2$, and $-COOR^2$ (where $R^2$ is an alkyl group, an aryl group, an amino group, or an alkoxy group)]
the ligand is immobilized on the base carrier via one or more types of active groups having reactivity to the ligand.

[2] The chromatography carrier according to [1], wherein the ligand is represented by the formula (1).
[3] The chromatography carrier according to [1] or [2], wherein Z in the formula (1) represents an alkylene group, a cycloalkylene group, -NR-, -O-, an arylene group, -S- or a combination thereof, each of which may have a substituent (where R represents a hydrogen atom, an alkyl group, an aryl group, an alkyl ester group, an aryl ester group, an alkyl ether group or an aryl ether group).
[4] The chromatography carrier according to [3], wherein Z in the formula (1) represents an alkylene group having 2 to 12 carbon atoms, a cycloalkylene group having 3 to 6 carbon atoms, - O-, a phenylene group or a combination thereof.
[5] The chromatography carrier according to [1], wherein the ligand is a polyallylamine having some amino groups modified, which includes a structural unit represented by the formula (2) and a structural unit represented by the formula (3), and in the formula (3), one of Y is an alkyl group having 1 to 12 carbon atoms, a phenyl group, or $-COR^2$ (where $R^2$ is an alkyl group having 1 to 12 carbon atoms or a phenyl group), and the other is a hydrogen atom.

[6] The chromatography carrier according to [1], wherein the ligand includes a group derived from one or more compounds selected from the group consisting of 1,2-diaminoethane, 1,3-diaminopropane, 1,4-diaminobutane, 2-methyl-1,3-propanediamine, 1,5-diaminopentane, 1,3-diaminopentane, 1,6-diaminohexane, 3,3'-diaminodipropylamine, 3,3'-diamino-N-methyldipropylamine, 2-methyl-1,5-diaminopentane, 1,4-diaminocyclohexane, 1,7-diaminoheptane, 1,8-diaminooctane, 4-(2-aminoethyl)cyclohexylamine, 1,9-diaminononane, 1,10-diaminodecane, 1,4-butanediol-bis(isopropylamine), 1,11-diaminoundecane, 1,12-diaminododecane, N,N-bis(3-aminopropyl)1,4-butanediamine, 6,6'-diaminodihexylamine, p-xylylenediamine, 4-picolylamine, and a polyallylamine having some amino groups modified, which includes a structural unit represented by the following formula (2') and a structural unit represented by the following formula (3'),

[Chemical Formula 2]

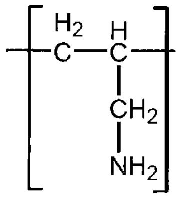
(2')

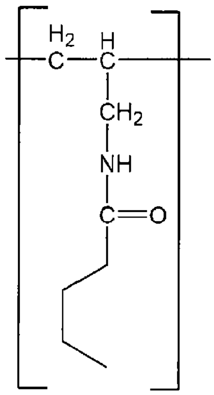
(3')

[7] The chromatography carrier according to any one of [1] to [6], wherein the active groups are selected from the group consisting of an epoxy group, a formyl group, and a vinyl sulfone group.
[8] The chromatography carrier according to any one of [1] to [7], wherein the porous particles are crosslinked cellulose.
[9] The chromatography carrier according to any one of [1] to [8], wherein the porous particles have an average pore diameter of 200 nm or more.
[9-1] The chromatography carrier according to any one of [1] to [9], wherein the porous particles have a volume average particle diameter of 30 $\mu m$ to 1000 $\mu m$.
[9-2] The chromatography carrier according to any one of [1] to [9-1], wherein a ligand density is 30 $\mu mol/g$ to 1500 $\mu mol/g$.
[10] A method for purifying an antibody, including: bringing a solution including an antibody and a host cell protein (HCP) into contact with the chromatography carrier according to any one of [1] to [9-2] to obtain an antibody solution with a reduced HCP content.
[11] The method according to [10], wherein the step of bringing the solution including the antibody and the host cell protein (HCP) into contact with the chromatography carrier is performed by passing the solution through a container containing the chromatography carrier in a flow-through mode.
[12] The method according to [10] or [11], wherein the antibody is a monoclonal antibody.
[13] The method according to any one of [10] to [12], wherein the HCP content in the antibody solution is 300 ppm or less.
[14] The method according to any one of [10] to [13], wherein the porous particles have an average pore diameter of 300 nm to 1500 nm, and the HCP content in the antibody solution is 100 ppm or less.
[15] The method according to any one of [10] to [14], wherein the antibody is purified with a recovery rate of 85% or more.

Effects of the Invention

According to the present invention, it is possible to provide a chromatography carrier capable of purifying a target substance with a high recovery rate, and a method for purifying an antibody using the same.

DESCRIPTION OF THE EMBODIMENTS

Hereinafter, embodiments of the present invention will be described in detail. It is noted that the materials, configurations, and the like described below are not intended to limit the present invention, and various modifications can be made within the scope of the spirit of the present invention.

1. Definition

The terms in this specification are used in the meanings generally used by those skilled in the art, unless specifically defined below.
In this specification, "Tris" refers to trishydroxymethylaminomethane.
In this specification, for example, when it is described as "20 mM Tris-HCl buffer solution (pH 7.0)+NaCl (6 mS/cm)", it refers to a solution in which 2.42 g (20 mM) of trishydroxymethylaminomethane is dissolved per 1 L of solution, adjusted to pH 7.0 with hydrochloric acid (HCl), and adjusted to an electrical conductivity of 6 mS/cm with sodium chloride (NaCl).
In this specification, "antibody" refers to an immunoglobulin molecule that is capable of specifically binding to a target such as carbohydrates, polynucleotides, lipids, or polypeptides through at least one antigen recognition site located in the variable region of the immunoglobulin molecule. When used in this specification, antibody includes not only intact (for example, full-length) polyclonal or monoclonal antibodies, but also other modified structures of immunoglobulin molecules including antigen recognition sites of required specificity, including antigen-binding fragments thereof (such as Fab, Fab', F(ab')2, and Fv), single chain (scFv), mutants thereof, fusion proteins including antibody portions, humanized antibodies, chimeric antibodies, bispecific antibodies, linear antibodies, single chain antibodies, multispecific antibodies (for example, bispecific antibodies), as well as glycosylation variants of antibodies, amino acid sequence variants of antibodies, and covalently modified antibodies. Antibody includes antibodies of any class such as IgD, IgE, IgG, IgA, or IgM (or subclasses thereof), and the antibody needs not be of any particular class. Immunoglobulins may be assigned to different classes according to the antibody amino acid sequence of the constant region of the heavy chain. There are five major classes of immunoglobulins: IgA, IgD, IgE, IgG, and IgM, some of which may be further classified into subclasses (isotypes), such as IgG1, IgG2, IgG3, IgG4, IgA1, and IgA2.
In this specification, "HCP" is a general term for proteins derived from host cells.
In this specification, "removal" refers to removing substances other than the target substance from the solution before purification. For example, the state in which HCP is removed refers to a state in which the HCP amount is reduced compared to the solution before contact with the chromatography carrier. This is because, for example, when a solution including HCP is brought into contact with a chromatography carrier, the HCP is retained on the chromatography carrier, resulting in a reduction in HCP amount in the recovered solution.
In this specification, "bind-elute mode" refers to a purification method in which the target substance is first bound to a chromatography carrier and then eluted and recovered. For example, in the case of purifying an antibody, the antibody is first bound to a chromatography carrier, and impurities pass through the column without binding to the chromatography carrier. Next, using a mobile phase having an appropriate salt concentration or pH, the antibody bound to the chromatography carrier is eluted into the mobile phase and recovered. Examples of the elution methods include a one-step elution method in which the antibody is eluted by passing a buffer solution having a specific salt concentration or pH that reduces the affinity between the antibody and the chromatography carrier, a stepwise method in which the antibody is eluted by changing the salt concentration or pH stepwise, and a gradient method in which the antibody is eluted by changing the salt concentration or pH continuously.
In this specification, "flow-through mode" refers to a purification method in which impurities are bound to a chromatography carrier, and the target substance flows and is recovered without binding to the chromatography carrier. For example, in the case of the target substance being an antibody and the impurity being host cell protein (HCP), HCP binds to the chromatography carrier, and the antibody flows through the column without binding to the chromatography carrier. In this case, the antibody may also bind to the chromatography carrier to some extent, but the antibody is purified by binding HCP more selectively to the chromatography carrier.

2. Chromatography Carrier

The chromatography carrier according to one embodiment of the present invention includes a base carrier including porous particles, and a ligand immobilized on the base carrier,

wherein the ligand is represented by the following formula (1),

-NH-Z-X (1)

(in the formula, Z represents a divalent hydrophobic group; X represents $NH_2$ or represents a heterocyclic group including a nitrogen atom)

the ligand is immobilized on the base carrier via one or more types of active groups having reactivity to the ligand.

Alternatively, in other embodiments, the ligand may be a polyallylamine having some amino groups modified or a salt thereof. The polyallylamine having some amino groups modified or a salt thereof includes a structural unit represented by the following formula (2) and a structural unit represented by the following formula (3) or formula (3a), and has a weight average molecular weight of 1000 to 500000.

[Chemical Formula 3]

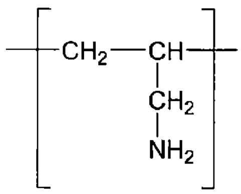

(2)

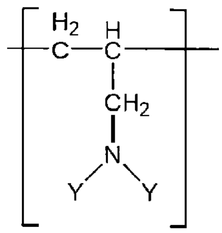

(3)

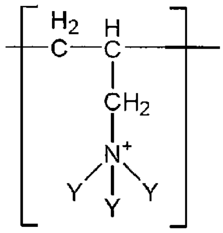

(3a)

[in formulas (3) and (3a), Y is each independently selected from the group consisting of a hydrogen atom, an alkyl group, an aryl group, $-COR^2$, and $-COOR^2$ (where $R^2$ is an alkyl group, an aryl group, an amino group, or an alkoxy group)]

The chromatography carrier according to the embodiment is a multimodal chromatography carrier having hydrophobic groups and amino groups in the ligand, and excels in the ability to separate the target substance and impurities. As a result, the target substance can be obtained with a high recovery rate and purity, and in other words, the chromatography carrier excels in the ability to remove impurities. The reason why using the ligand having the structure as described above achieves such effects is not clear, but the amino groups of the ligand can form hydrogen bonds in addition to electrostatic interaction, and can interact with impurities through hydrophobic interaction of hydrophobic groups. Therefore, it is presumed that multiple interactions can complement each other to interact with impurities.

The target substance is not particularly limited, but examples include proteins such as antibodies, peptides, nucleic acids, plasmids, viruses, virus-like particles, extracellular vesicles, and the like. Examples of antibodies, as defined above, include polyclonal antibodies, monoclonal antibodies, antigen-binding fragments thereof (such as Fab, Fab', F(ab')2, and Fv), single chain (scFv), mutants thereof, fusion proteins including antibody portions, humanized antibodies, chimeric antibodies, bispecific antibodies, linear antibodies, single chain antibodies, multispecific antibodies (for example, bispecific antibodies), and glycosylation variants of antibodies, amino acid sequence variants of antibodies, covalently modified antibodies, and the like, with monoclonal antibodies or antigen-binding fragments thereof being preferable.

Impurities are not particularly limited, but examples include antibody aggregates, host cell protein (HCP), host-derived DNA, leaked protein A, viruses, endotoxins, medium components, nutrients, and the like. Among these, HCP is a mixture of multiple types of proteins and has been attracting attention in recent years. HCP can not only be recognized by the immune system as a foreign antigen itself, but some HCPs also have enzymatic activity that degrades the target substance. Therefore, there is a demand for effectively separating HCP to obtain the target substance with a low HCP content. Particularly, in the field of antibody pharmaceuticals, effectively removing HCP that may affect the quality and safety of pharmaceuticals is greatly beneficial.

In the case of using the chromatography carrier according to the embodiment for antibody purification, it is possible to obtain an antibody with a high recovery rate, and the HCP amount included in the obtained antibody solution is low. That is, it can be said that the chromatography carrier according to the embodiment excels particularly in the ability to separate antibody and HCP.

(1) Base Carrier

The chromatography carrier generally has a structure with a ligand bound to a base carrier. The base carrier used in the chromatography carrier according to the embodiment includes porous particles, and the porous particles may be modified with active groups for introducing the ligand, as described later. The base carrier used is not limited as long as the porous particles are modified with active groups, and preferable examples include polysaccharides and derivatives thereof such as agarose, dextran, starch, cellulose, pullulan, chitin, chitosan, cellulose triacetate, and cellulose diacetate; organic polymers such as polyacrylamide, polymethacrylamide, polyacrylate, polymethacrylate, polyalkyl vinyl ether, and polyvinyl alcohol, and the like. It is preferable that the porous particles form a crosslinked structure from the viewpoint of ensuring mechanical strength. Among these, it is more preferable to use crosslinked cellulose particles in which the skeleton of cellulose particles is reinforced by crosslinking reaction.

The crosslinked cellulose particles are not particularly limited as long as the crosslinked cellulose particles can be used as a base carrier for the chromatography carrier. The cellulose used as a raw material may be crystalline cellulose or amorphous cellulose.

Examples of crosslinked cellulose that can be suitably used include the porous cellulose gel disclosed in Japanese Patent Application Laid-Open No. 2009-242770. The porous cellulose gel disclosed in the publication is obtained by a method including a step of continuously dropping or dividedly adding, over 3 hours or more, a crosslinking agent in an amount 4 to 12 times the number of moles of cellulose monomer and an alkali in an amount 0.1 to 1.5 times the number of moles of the crosslinking agent to a suspension of uncrosslinked cellulose particles in the presence of at least one type of inorganic salt selected from the group consisting of hydrochlorides, sulfates, phosphates, and borates in an amount 6 to 20 times the number of moles of cellulose monomer. The crosslinked cellulose particles obtained in this manner have high mechanical strength, may be used under chromatography conditions with a high flow rate, and can provide a chromatography carrier with high productivity. Here, "cellulose monomer" means glucose units that are structural units of cellulose. Further, the number of moles of cellulose monomer (that is, degree of polymerization) is calculated based on the amount obtained by subtracting moisture from glucose units (that is, dry weight of cellulose) (with molecular weight 162 as 1 mole). The crosslinking agent can be appropriately selected and used from crosslinking agents commonly used in the field, and examples include epichlorohydrin, epibromohydrin, dichlorohydrin, ethylene glycol diglycidyl ether, 1,4-butanediol diglycidyl ether, polyethylene glycol diglycidyl ether, glycerol polyglycidyl ether, and the like, with epichlorohydrin, epibromohydrin, or glycerol glycidyl ether being preferable.

The shape of the porous particles is not particularly limited, but spherical particles are preferable because spherical particles have high mechanical strength, excel in gel sedimentation properties, and can create a uniform packed bed. In this case, the sphericity of the porous particles is preferably 0.8 to 1.0. Here, "sphericity" means the minor diameter/major diameter of the porous particles.

Spherical cellulose particles can be easily obtained, for example, by dissolving and regenerating crystalline cellulose or cellulose composed of crystalline regions and amorphous regions. Methods for producing spherical cellulose particles include, for example, methods via acetate esters described in Japanese Patent Publication No. 55-39565, Japanese Patent Publication No. 55-40618, Japanese Patent Application Laid-Open No. 2023-037724, and the like; methods for producing from solutions including calcium thiocyanate salt described in Japanese Patent Publication No. 63-62252 and the like; methods for producing from solutions including paraformaldehyde and dimethyl sulfoxide described in Japanese Patent Application Laid-Open No. 59-38203 and the like; methods for producing from cellulose solutions in which cellulose is dissolved in lithium chloride-containing amide described in Japanese Patent No. 3663666, and the like. Further, spherical crosslinked cellulose particles can be obtained by crosslinking spherical cellulose particles.

The particle diameter of the porous particles is preferably 10 μm to 500 μm, and particularly preferably 50 μm to 150 μm. Further, the average particle diameter is preferably 30 μm to 1000 μm, more preferably 40 μm to 200 μm, and particularly preferably 50 μm to 100 μm. Here, "particle diameter" means the actual measured value of the particle diameter of each porous particle, and "average particle diameter" means the average value calculated based on the particle diameter, and particularly means the volume average particle diameter.

In this specification, the particle diameter and the average particle diameter of the porous particles can be measured, for example, using a laser diffraction/scattering particle diameter distribution measurement apparatus. In this apparatus, a particle group is irradiated with laser light, the particle size distribution is determined from the intensity distribution pattern of diffraction/scattering light emitted therefrom, and the particle diameter and average particle diameter are calculated based on this. As a specific measurement apparatus, a laser diffraction/scattering particle diameter distribution measurement apparatus LA-960 (Horiba, Ltd.) and the like can be used.

Alternatively, the particle diameter can also be measured using images captured with an optical microscope. Specifically, the particle diameter on the image is measured using calipers or the like, and the original particle diameter is determined from the magnification. Then, the average particle diameter is calculated by the following formula from the value of each particle diameter obtained from the optical microscope image.

$$\text{Volume average particle diameter (MV)}=\Sigma(nd^4)/\Sigma(nd^3)$$

[In the formula, d represents the value of the particle diameter of each particle obtained from the optical microscope image, and n represents the number of measured particles.]

The porosity of the porous particles can be characterized by pore size characteristics. One indicator showing pore size is the retention time of a standard substance when packing porous particles in a column. Here, the retention time refers to the time required from injection of a sample into the column to the elution peak. Pore size affects the physical strength of particles and the diffusivity of the target substance to be purified within the porous particles. Accordingly, differences occur in the flow rate of liquid passing through the porous particles and the dynamic adsorption capacity of the porous particles depending on pore size. Therefore, it is required to design porous particles with a pore size appropriate for the intended purpose.

The average pore diameter ($d_{pore}$) of the porous particles is preferably 80 nm or more (for example, 80 nm to 5000 nm, 80 nm to 3000 nm, 80 nm to 1500 nm), more preferably 200 nm or more (for example, 200 nm to 5000 nm, 200 nm to 3000 nm, 200 nm to 1500 nm), even more preferably 300 nm or more (for example, 300 nm to 5000 nm, 300 nm to 3000 nm, 300 nm to 1500 nm), and particularly preferably 700 nm or more (for example, 700 nm to 5000 nm, 700 nm to 3000 nm, 700 nm to 1500 nm). Alternatively, the average pore diameter may be 500 nm or more (for example, 500 nm to 5000 nm, 500 nm to 3000 nm, 500 nm to 1500 nm). Here, the average pore diameter means a value obtained from the gel partition coefficient Kav calculated using standard polyethylene oxide, and is specifically calculated by the method described in Examples described later.

By using porous particles having an average pore diameter in the above-mentioned range as the base carrier, the base carrier has high adsorption capacity for the target substance or impurities with large molecular sizes, or aggregates of impurities. In particular, it is preferable to use cellulose particles having the above-mentioned average pore diameter, more preferably crosslinked cellulose particles having the above-mentioned average pore diameter.

(2) Ligand

The chromatography carrier according to the embodiment includes a ligand immobilized on the base carrier, and in one embodiment, the ligand has a structure represented by the following formula (1):

-NH-Z-X (1)

(in the formula, Z represents a divalent hydrophobic group; X represents $NH_2$ or represents a heterocyclic group including a nitrogen atom).

In formula (1), the divalent hydrophobic group represented by Z is not particularly limited as long as the divalent hydrophobic group is a group that exhibits hydrophobicity. Z as a whole may exhibit hydrophobicity, or a part of Z may exhibit hydrophobicity. Examples of such Z include an alkylene group, a cycloalkylene group, -NR-, -O-, an arylene group, -S- or a combination thereof, each of which may have a substituent (where R represents a hydrogen atom, an alkyl group, an aryl group, an alkyl ester group, an aryl ester group, an alkyl ether group or an aryl ether group). More preferably, Z is an alkylene group having 2 to 12 carbon atoms, a cycloalkylene group having 3 to 6 carbon atoms, -O-, a phenylene group or a combination thereof.

The substituent that Z may have is not limited as long as the hydrophobicity of Z is not inhibited, and examples include an alkyl group, a cycloalkyl group, an aryl group, an alkyl ether group, an aryl ether group, an alkylthioether group, an arylthioether group, and the like.

In X of formula (1), examples of the heterocyclic group including a nitrogen atom include a pyridyl group, a pyrrole group, an imidazole group, an indole group, a triazine group, a quinoline group, and the like. It is particularly preferable that X is $NH_2$.

In another embodiment, the ligand is a polyallylamine having some amino groups modified or a salt thereof. The polyallylamine having some amino groups modified or a salt thereof includes a structural unit represented by the following formula (2) and a structural unit represented by the following formula (3) or formula (3a):

[Chemical Formula 4]

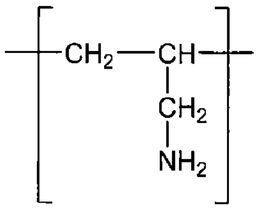
(2)

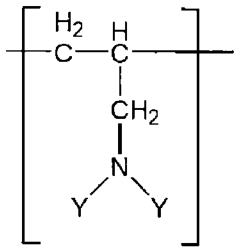
(3)

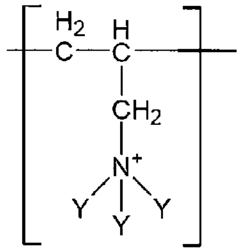
(3a)

[in formulas (3) and (3a), Y is each independently selected from the group consisting of a hydrogen atom, an alkyl group, an aryl group, $-COR^2$, and $-COOR^2$ (where $R^2$ is an alkyl group, an aryl group, an amino group, or an alkoxy group)].

Y in formula (3) may further have a substituent. The substituent that Y may have is not limited, but examples include the groups described above as the substituent that Z in formula (1) may have. Y is preferably a hydrogen atom, a phenyl group, an alkyl group having 1 to 12 carbon atoms, or $-COR^2$, and more preferably a hydrogen atom, an alkyl group having 1 to 4 carbon atoms, or $-COR^2$ ($R^2$ is an alkyl group having 1 to 4 carbon atoms).

The polyallylamine having some amino groups modified or a salt thereof may be composed of a structural unit represented by formula (2) and a structural unit represented by formula (3) or formula (3a), and may include other optional structural units.

In the case where the ligand is a salt of the polyallylamine having some amino groups modified, the type of salt is not particularly limited, but examples include hydrochloride, phosphate, sulfate, and acetate. The counter ion for $N^+$ in the above formula (3a) can be appropriately selected according to the structure of formula (3a). Examples of the counter ion include, but are not limited to, $Cl^-$, $SO_4^{2-}$, $HSO_4^-$, $PO_4^{3-}$, $HPO_4^{2-}$, $H_2PO_4^-$, $COO^-$, $C_6H_5O_7^{3-}$, $C_6H_6O_7^{2-}$, $C_6H_7O_7^-$, $BO_3^{3-}$, $HBO_3^{2-}$, $H_2BO_3^-$, and the like.

The weight average molecular weight of the polyallylamine having some amino groups modified or a salt thereof is 1000 to 500000, preferably 1500 to 50000, and more preferably 10000 to 20000.

Particularly preferable ligands include ligands derived from the compounds shown in Table 1 below.

[Table 1-1]

| Table 1 | | |
|---|---|---|
| | Ligand compound | Structure |
| 1 | 1,2-diaminoethane | 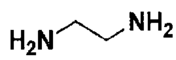 |
| 2 | 1,3-diaminopropane | 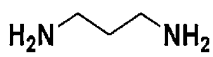 |
| 3 | 1,4-diaminobutane | 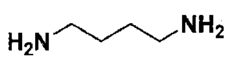 |
| 4 | 2-methyl-1,3-propanediamine | 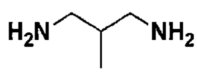 |
| 5 | 1,5-diaminopentane | 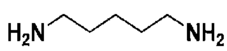 |

(continued)

| Table 1 | | |
|---|---|---|
| | Ligand compound | Structure |
| 6 | 1,3-diaminopentane | 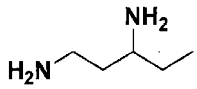 |
| 7 | 1,6-diaminohexane | 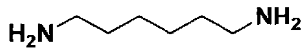 |
| 8 | 3,3'-diaminodipropylamine | 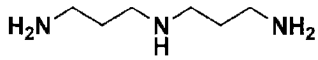 |
| 9 | 3,3'-diamino-N-methyldipropylamine | 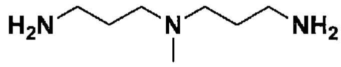 |
| 10 | 2-methyl-1,5-Diaminopentane | 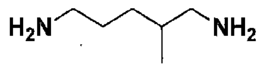 |
| 11 | 1,4-diaminocyclohexane | 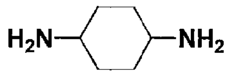 |
| 12 | 1,7-diaminoheptane | 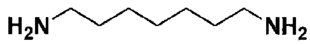 |
| 13 | 1,8-diaminooctane | 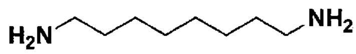 |
| 14 | 4-(2-aminoethyl)cyclohexylamine | 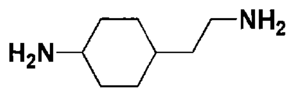 |
| 15 | 1,9-diaminononane | 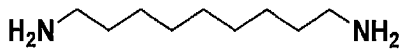 |
| 16 | 1,10-diaminodecane | 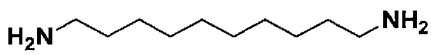 |
| 17 | 1,4-butanediol-bis(isopropylamine) | 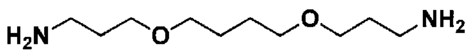 |
| 18 | 1,11-diaminoundecane | 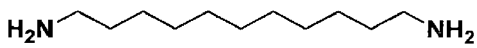 |

[Table 1-2]

| Table 1 (continued) | | |
|---|---|---|
| 19 | 1,12-diaminododecane | 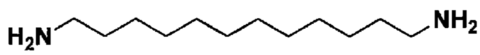 |
| 20 | N,N-bis(3-aminopropyl) 1,4-Butanediamine | 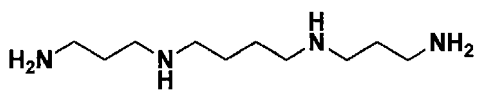 |
| 21 | 6,6'-diaminodihexylamine | 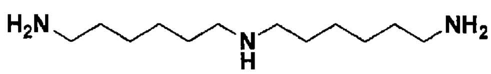 |
| 22 | p-xylylenediamine | 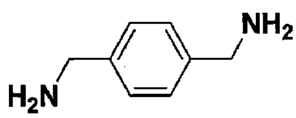 |

(continued)

| Table 1 (continued) | | |
|---|---|---|
| 23 | 4-picolylamine | 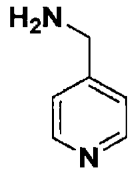 |

It is also preferable to use a ligand derived from a polyallylamine, for example, a ligand derived from a polyallylamine having some amino groups modified, which includes or is composed of a structural unit represented by the following formula (2') and a structural unit represented by the following formula (3').

[Chemical Formula 5]

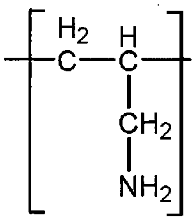

(2')

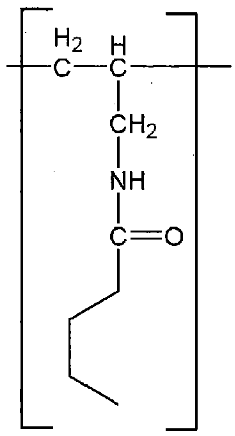

(3')

The method for binding the ligand to the base carrier is not particularly limited, but the ligand can be bound via one or more active groups that have reactivity to amino groups in the ligand. For example, the base carrier can be modified in advance with active groups, and the ligand can be bound thereto. Examples of such active groups include an N-hydroxysuccinimide group, an epoxy group, a formyl group, a vinyl sulfone group, an azlactone, and the like, with an epoxy group, a formyl group, or a vinyl sulfone group being preferable.

The ligand density (amount of ligand bound per 1 g of base carrier) is preferably 30 μmol/g to 1500 μmol/g, more preferably 70 μmol/g to 750 μmol/g, and particularly preferably 140 μmol/g to 360 μmol/g or 170 μmol/g to 310 μmol/g. Alternatively, the ligand density may be 140 μmol/g to 600 μmol/g or 150 μmol/g to 550 μmol/g. By setting such ligand density, a chromatography carrier can be obtained that maintains high adsorption capacity for the target substance or impurities while also having excellent washability and being capable of repeated use.

(3) Applications

The chromatography carrier according to the embodiment can be used in separation and purification of various target substances.

In purification, the chromatography carrier is first packed into a column, but the packing mode is not particularly limited. Next, a sample solution including the target substance is brought into contact with the chromatography carrier, thereby separating the target substance from impurities. Specifically, the target substance can be purified by packing the above-described chromatography carrier into a column, flowing the sample solution therethrough, and selectively adsorbing either the target substance or impurities to the chromatography carrier. Alternatively, the target substance can also be purified by adsorbing both the target substance and impurities to the chromatography carrier and utilizing the difference in affinity to the chromatography carrier by changing elution conditions (for example, salt concentration) stepwise or continuously.

The difference in affinity between the target substance and impurities for the chromatography carrier is utilized in

the setting of chromatography conditions. For example, conditions are set considering differences in carrier structure (ligand type, ligand density, ligand orientation, particle diameter, pore diameter, base matrix composition, and the like) and physicochemical properties of the target substance and impurities (isoelectric point, charge, hydrophobicity, molecular structure, three-dimensional structure, and the like). Conditions can be adjusted to perform chromatography in a bind-elute mode or a flow-through mode.

Components that can be included in the buffer solution used for the sample solution, column washing, elution, etc. are not particularly limited as long as the components have buffering ability, but examples include 1 mmol/L to 300 mmol/L of phosphate, citrate, acetate, succinate, maleate, borate, tris(base), HEPES, MES, PIPES, MOPS, TES, Tricine, and the like. The above salts can also be used in combination with other salts such as sodium chloride, potassium chloride, calcium chloride, sodium citrate, sodium sulfate, and ammonium sulfate. Furthermore, the buffer solution may include amino acids such as glycine, alanine, arginine, serine, threonine, glutamic acid, aspartic acid, and histidine, sugars such as glucose, sucrose, lactose, and sialic acid, or derivatives thereof.

The pH of the buffer solution is preferably in the range of 2 to 9, and more preferably in the range of 3 to 8.

The linear velocity of the buffer solution is preferably in the range of 20 cm/h to 1000 cm/h.

According to the method of the embodiment, the target substance can be purified with a recovery rate of preferably 85% or more, more preferably 90% or more, and particularly preferably 95% or more. Here, the recovery rate means the ratio of the recovered amount of the target substance after purification to the amount of the target substance loaded onto the chromatography carrier (that is, the amount of the target substance in the sample solution before purification).

The chromatography carrier according to the embodiment can be suitably used, for example, in protein purification, particularly in antibody purification. As described above, since the chromatography carrier particularly excels in the ability to separate antibody and HCP, using the chromatography carrier in antibody purification can obtain an antibody solution with a low HCP content.

Therefore, according to one embodiment of the present invention, a method for purifying an antibody is provided, which includes bringing a solution including an antibody and a host cell protein (HCP) into contact with the above-described chromatography carrier to obtain an antibody solution with a reduced HCP content. Here, the antibody is preferably a monoclonal antibody. The step of bringing the solution including the above-described antibody and host cell protein (HCP) into contact with the chromatography carrier is preferably performed by passing the sample solution through a container containing the chromatography carrier in a flow-through mode.

The antibody loading amount per unit volume of the chromatography carrier is, for example, 10 g/L to 2000 g/L, 100 g/L to 1500 g/L, or 200 g/L to 1500 g/L. In the case of using the chromatography carrier of the embodiment, a purified antibody can be obtained with a high recovery rate and/or a low impurity amount (particularly low HCP amount) even if the antibody loading amount per unit volume of the chromatography carrier is 200 g/L or more, 500 g/L or more, or 1000 g/L or more. In other words, using the chromatography carrier of the embodiment makes it possible to obtain a purified antibody with a high recovery rate and/or a low impurity amount even if a large amount of antibody is loaded onto the chromatography carrier at once, which improves the efficiency of antibody purification.

According to the method of the embodiment, the HCP amount included in the purified antibody solution (recovery fraction) is 300 ppm or less (0 ppm to 300 ppm), preferably 100 ppm or less (0 ppm to 100 ppm), more preferably 30 ppm or less (0 ppm to 30 ppm), and particularly preferably 15 ppm or less (0 ppm to 15 ppm) or 10 ppm or less (0 ppm to 10 ppm). It is noted that the HCP amount is a value calculated from the formula {HCP amount in purified antibody solution (ng)/antibody amount in purified antibody solution (mg)}.

The purification method according to the embodiment may be performed in combination with other purification methods. Methods suitable for antibody purification, for example, can be used as other purification methods, and examples include chromatography, activated carbon treatment, alcohol fractionation, precipitate removal, salting out, buffer exchange, concentration, dilution, filtration, virus inactivation, virus removal, and the like. One or multiple methods may be selected as other purification methods, and may be performed before or after the purification method according to the embodiment.

Further, in the field of monoclonal antibody production, generally cation exchangers are used in a bind-elute mode and anion exchangers are used in a flow-through mode, but with use of the chromatography carrier of the embodiment, it is also applicable to methods using cation exchangers in a flow-through mode.

In the case of other purification methods being chromatography, examples of the carrier or membrane used include heparin carriers and affinity carriers such as protein A, affinity membranes, cation exchange carriers, cation exchange membranes, anion exchange carriers, anion exchange membranes, gel filtration carriers, hydrophobic interaction carriers, reverse phase carriers, hydroxyapatite carriers, fluoroapatite carriers, sulfated cellulose carriers, sulfated agarose carriers, multimodal carriers, and the like.

## Examples

Hereinafter, the present invention will be described in detail based on examples, but the content of the present invention is not limited thereby.

<Example 1: Production of Cellulose Particles>

[Production of Crosslinked Cellulose Particles (Hydrated) A]

(Granulation Process)

(1) To 100 g of a 60 wt% aqueous solution of calcium thiocyanate, 6.4 g of crystalline cellulose (manufactured by Asahi Kasei Chemicals Corporation, trade name: Ceolus PH101) was added and dissolved by heating to 110°C to 120°C.
(2) To this solution, 6 g of sorbitan monooleate was added as a surfactant. This was dropped into 480 mL of o-dichlorobenzene preheated to 130°C to 140°C and stirred at 200 rpm to 300 rpm to obtain a dispersion.
(3) Next, the dispersion was cooled to 40°C. This was poured into 190 mL of methanol to obtain a suspension of particles.
(4) The obtained suspension was filtered and separated to recover particles, and the particles were washed with 190 mL of methanol. This washing operation was performed several times.
(5) Furthermore, the particles were washed with a large amount of water to obtain spherical cellulose particles.
(6) The obtained spherical cellulose particles were passed through sieves with openings of 125 μm and 53 μm to obtain spherical cellulose particles with a particle diameter of 53 μm to 125 μm.

(Crosslinking Process)

(1) To 100 g of the spherical cellulose particles (hydrated) obtained above, 121 g of pure water was added and heated while stirring. When the temperature reached 30°C, 3.3 g of 45 wt% NaOH aqueous solution and 0.5 g of $NaBH_4$ were added, and heating and stirring were further performed. Here, the initial alkali concentration was 0.69% (w/w).
(2) After 30 minutes, 60 g of $Na_2SO_4$ was added to the reaction solution and dissolved. From the point when the temperature of the mixture reached 50°C, stirring was continued for an additional 2 hours while maintaining the temperature at 50°C.
(3) While continuing to stir the mixture at 50°C, 48 g of 45 wt% NaOH aqueous solution and 50 g of epichlorohydrin were each divided into 24 equal portions and added every 15 minutes over approximately 6 hours.
(4) After completion of the addition, this mixture was reacted at a temperature of 50°C for 16 hours.
(5) After cooling the reaction mixture to a temperature of 40°C or below, 2.6 g of acetic acid was added for neutralization.
(6) The reaction mixture was filtered to recover the cellulose particles, and the cellulose particles were filtered and washed with pure water to obtain crosslinked cellulose particles A.

[Production of Crosslinked Cellulose Particles (Hydrated) B]

(Granulation Process)

(1) 100 g of cellulose acetate (L-20, manufactured by Daicel Corporation) was added to a mixed solvent of 391 g of benzyl alcohol, 276 g of 1-hexanol, and 3.5 g of polypropylene glycol (Wako Pure Chemical reagent, diol type, average molecular weight 1000) and stirred.
(2) The temperature was raised and the mixture was stirred at 120°C for 4 hours to dissolve the cellulose acetate, and a transparent cellulose acetate solution was obtained.
(3) 2 g of PVA (JP-18E, manufactured by Japan Vam & Poval Co., Ltd.) and 30 g of sodium carboxymethylcellulose (CMC1140, manufactured by Daicel Miraizu Ltd.) were added to 2300 g of pure water saturated with the mixed solvent, and the temperature was raised and the mixture was stirred and dissolved at 80°C for 1 hour or more to obtain a dispersion medium.

(4) 770 g of the cellulose acetate solution was quickly poured into 2300 g of the dispersion medium, and the mixture was stirred at 80°C to obtain a dispersion system. Subsequently, this dispersion system was cooled to 35°C to obtain spherical cellulose acetate particles.
(5) The obtained cellulose acetate particles were thoroughly washed with a large amount of water, then with methanol, and then washed again with pure water.

(Saponification Process)

(1) 630 g-wet of the obtained cellulose acetate particles (water content 6.86) were added to a mixed solution of 1072 g of pure water and 242 g of methanol, and the mixture was stirred at a temperature of 35°C for 30 minutes. The water content of the cellulose particles was calculated by "wet weight of cellulose particles/dry weight".
(2) 201 g of 20% NaOH was added, and the mixture was stirred and reacted at 35°C for 2 hours to perform saponification.
(3) After cooling to 30°C or below and neutralizing with acetic acid, the mixture was thoroughly washed with pure water.
(4) The obtained saponified cellulose spherical particles were passed through sieves with openings of 150 μm and 45 μm to obtain saponified cellulose particles with a particle diameter of 45 μm to 150 μm.

(Crosslinking Process)

(1) 96.4 g-wet of the obtained saponified cellulose particles (water content 7.93) were dispersed in 194 g of pure water, and then 78.6 g of $Na_2SO_4$ was dissolved.
(2) After stirring at a temperature of 50°C for 30 minutes, 4.0 g of 48% NaOH and 0.69 g of $NaBH_4$ were added, and the mixture was dissolved and reacted over 30 minutes.
(3) After dissolution, 36.4 mL of 48% NaOH solution and 62 g of epichlorohydrin were each divided into 8 equal portions and added every 30 minutes over approximately 4 hours.
(4) After completion of the addition, the mixture was reacted at a temperature of 50°C for 16 hours. The temperature was cooled to 30°C or below, and acetic acid was added for neutralization.
(5) The reaction mixture was filtered to recover the gel, which was then washed by filtration with pure water to obtain crosslinked porous particles.

[Production of Crosslinked Cellulose Particles (Hydrated) C]

Particles were produced by the same method as the crosslinked cellulose particles B described above, except that the amount of polypropylene glycol used in the granulation process was changed to 7.0 g.

[Production of Crosslinked Cellulose Particles (Hydrated) D]

Particles were produced by the same method as the crosslinked cellulose particles B described above, except that the amount of polypropylene glycol used in the granulation process was changed to 10.5 g.

[Production of Crosslinked Cellulose Particles (Hydrated) E]

Particles were produced by the same method as the crosslinked cellulose particles B described above, except that the amount of polypropylene glycol used in the granulation process was changed to 14.0 g.

[Production of Crosslinked Cellulose Particles (Hydrated) F]

Particles were produced by the same method as the crosslinked cellulose particles B described above, except that the amount of polypropylene glycol used in the granulation process was changed to 8.5 g.

<Example 2: Analysis of Crosslinked Cellulose Particles>

The following analysis was performed on the crosslinked cellulose particles A to F produced in Example 1.

(Measurement of Average Particle Diameter)

The volume average particle diameter was measured using a laser diffraction/scattering particle diameter distribution measurement apparatus LA-960 (manufactured by Horiba, Ltd.).

(Elution Time of Standard Polyethylene Oxide)

The crosslinked cellulose particles A to F obtained above were packed into a stainless steel column (manufactured by Tosoh Corporation) with an inner diameter of 0.78 cm and a length of 30 cm, and then the retention times of TSKgel standard polyethylene oxide (SE-70, SE-30, SE-15, SE-8, SE-5 manufactured by Tosoh Corporation) with molecular weights of $4.42\times10^4$ to $5.8\times10^5$ were measured. The packing method involved dispersing the particles in pure water to form a slurry, packing the particles into the column, and then flowing pure water at a flow rate of 0.4 ml/min for 1 hour or more to achieve consolidation. Pure water was used as the mobile phase for measurement, and 10 $\mu$L of polyethylene oxide prepared to 5.0 mg/mL was injected. The value at which the RI detection intensity reached the maximum was taken as the elution peak, and that time was taken as the retention time. The apparatus used for measurement was as follows.
Apparatus: 1260 Infinity HPLC system (manufactured by Agilent Technologies)

(Measurement of Average Pore Diameter $d_{pore}$)

The average pore diameter can be calculated from the gel partition coefficient $K_{av}$ calculated using standard polyethylene oxide, and the average pore diameter and gel partition coefficient $K_{av}$ have a relationship represented by the following formula (where intraparticle porosity=$\varepsilon_p$, viscosity radius of standard polyethylene oxide molecule=$r_m$, and average pore radius $r_{pore}$).

$$K_{av}=\varepsilon_p(1-r_m/r_{pore})^2$$

The gel partition coefficient $K_{av}$ was calculated by the following formula from the relationship between the retention volume and column volume, using standard polyethylene oxide (SE-70, SE-30, SE-15, SE-8, SE-5 manufactured by Tosoh Corporation) with weight average molecular weights of $4.42\times10^4$ to $5.8\times10^5$ Da as samples. Pure water was used as the mobile phase.

$$K_{av}=(V_e-V_0)/(V_t-V_0)$$

[In the formula, $V_e$ represents the retention volume of the sample (mL), $V_t$ represents the empty column volume (mL), and $V_0$ represents the interparticle void volume (mL), which was determined by $0.4\times V_t$.]
By plotting the viscosity radius of multiple standard polyethylene oxide molecules against the 0.5 power of the $K_{av}$ measured values, the average pore radius $r_{pore}$ was calculated from the slope of the approximation curve, and $d_{pore}$ was taken as twice this value.
The analysis results of the crosslinked cellulose particles are shown in Table 2.

[Table 2]

Table 2

| Crosslinked cellulose particles | Average particle diameter ($\mu$m) | Polyethylene oxide elution time (min) | | | | | $d_{pore}$ (nm) |
|---|---|---|---|---|---|---|---|
| | | SE-70 | SE-30 | SE-15 | SE-8 | SE-5 | |
| Crosslinked cellulose particles A | 85 | 15.5 | 18.2 | 21.8 | 23.4 | 27.0 | 86 |
| Crosslinked cellulose particles B | 96 | 22.2 | 24.8 | 26.3 | 27.5 | 29.0 | 210 |
| Crosslinked cellulose particles C | 90 | 25.4 | 27.1 | 28.1 | 29.1 | 29.4 | 360 |
| Crosslinked cellulose particles D | 97 | 27.9 | 30.0 | 30.8 | 29.7 | 30.5 | 710 |
| Crosslinked cellulose particles E | 88 | 28.1 | 28.5 | 28.9 | 29.0 | 29.5 | 1104 |

(continued)

| Crosslinked cellulose particles | Average particle diameter (μm) | Polyethylene oxide elution time (min) | | | | | $d_{pore}$ (nm) |
|---|---|---|---|---|---|---|---|
| | | SE-70 | SE-30 | SE-15 | SE-8 | SE-5 | |
| Crosslinked cellulose particles F | 83 | 26.1 | 27.2 | 27.5 | 28.1 | 28.8 | 575 |

<Example 3: Modification of Crosslinked Cellulose Particles with Active Groups and Ligand>

(1) Epoxidized Cellulose Particles

[Epoxidation of Cellulose Particles]

3000 g of the crosslinked cellulose particles A obtained in Example 1 and 1952 g of pure water were added to a 10 L SUS container to form a slurry. Next, 1764 g of epichlorohydrin was added. After heating to 28°C, 1655 g of 48.7% sodium hydroxide aqueous solution was added dropwise over 2 hours so that the liquid temperature did not exceed 30°C. After completion of the dropwise addition, stirring was continued for an additional 3 hours at 30°C. Next, 145 g of acetic acid was added and stirred for 10 minutes. After reaction completion, the wet particles were recovered by filtration, and the recovered wet particles were washed 16 times with 6 L of pure water to obtain the target epoxidized cellulose particles.

[Immobilization of Ligand to Epoxidized Cellulose Particles-1]

9.8 g of each ligand compound was dissolved in 16.5 of pure water and heated to 30°C. Next, 15.0 g of the epoxidized cellulose particles obtained above was added and stirred at 30°C for 18 hours. After reaction completion, the wet particles were recovered by filtration, and the recovered particles were washed 6 times with 50 mL of pure water to obtain the target products (carriers 1 to 5). The density of each ligand bound to the epoxidized cellulose particles is shown in Table 3. Here, ligand density (μmol/g) means the amount of ligand bound per 1 g of base carrier.

[Table 3]

Table 3

| Carrier | Base carrier | Active group | Ligand compound | Ligand density (μmol/g) |
|---|---|---|---|---|
| Carrier 1 | Crosslinked cellulose particles A | Epoxy group | 1,2-diaminoethane | 279 |
| Carrier 2 | Crosslinked cellulose particles A | Epoxy group | 1,4-diaminobutane | 304 |
| Carrier 3 | Crosslinked cellulose particles A | Epoxy group | 1,6-diaminohexane | 279 |
| Carrier 4 | Crosslinked cellulose particles A | Epoxy group | 1,8-diaminooctane | 282 |
| Carrier 5 | Crosslinked cellulose particles A | Epoxy group | 1,10-diaminodecane | 239 |

[Immobilization of Ligand to Epoxidized Cellulose Particles-2]

The ligand immobilization reaction was performed by the same method as above, except that the solvent for dissolving each ligand compound was changed to 13.1 g of methanol, and washing with 50 mL of methanol 3 times was added after reaction completion, to obtain the target products (carriers 6 to 9). The density of each ligand bound to the epoxidized cellulose particles is shown in Table 4.

[Table 4]

Table 4

| Carrier | Base carrier | Active group | Ligand compound | Ligand density (μmol/g) |
|---|---|---|---|---|
| Carrier 6 | Crosslinked cellulose particles A | Epoxy group | 1,12-diaminododecane | 207 |
| Carrier 7 | Crosslinked cellulose particles A | Epoxy group | 1,4-butanediol-bis(isopropylamine) | 232 |
| Carrier 8 | Crosslinked cellulose particles A | Epoxy group | p-xylylenediamine | 307 |
| Carrier 9 | Crosslinked cellulose particles A | Epoxy group | 4-picolylamine | 285 |

[Immobilization of Ligand to Epoxidized Cellulose Particles-3]

Epoxidized cellulose particles were obtained by the same method as carrier 1 above, except that the crosslinked cellulose particles E obtained in Example 1 were used.

117.4 g of polyallylamine 15.3% aqueous solutionPAA-15C (Nittobo Medical Co., Ltd.) with a polymerization average molecular weight of 15000 was mixed with 30.5 g of pure water. Next, 40.0 g of the epoxidized cellulose particles obtained above were added, the temperature was raised to 45°C, and the mixture was stirred for 18 hours. After reaction completion, the wet particles were recovered by filtration, and the recovered particles were washed 6 times with 120 mL of pure water to obtain an intermediate. 20.0 g of the obtained intermediate was suspended in 28.5 g of methanol, 0.36 g of valeric anhydride was added, and the mixture was stirred at 30°C for 16 hours (a ligand in which valeryl groups were added to some of the amino groups of PAA-15C was obtained). After reaction completion, the wet particles were recovered by filtration, and the recovered particles were washed once with 60 mL of methanol, 5 times with 60 mL of pure water, once with 20 mL of 0.5 mol/L sodium hydroxide solution, and 6 times with 60 mL of pure water to obtain the target product (carrier 23). The ligand density of carrier 23 is shown in Table 5. The ligand density of carrier 23 was calculated from the number of moles of amino groups in the ligand.

[Table 5]

Table 5

| Carrier | Base carrier | Active group | Ligand compound | Ligand density (μmol/g) |
|---|---|---|---|---|
| Carrier 23 | Crosslinked cellulose particles E | Epoxy group | Valeryl group-added PAA-15C | 510 |

(2) Formylated Cellulose Particles

[Formylation of Cellulose Particles]

100 g of the crosslinked cellulose particles A obtained in Example 1 and 248 g of pure water were added to a glass container to form a slurry. After heating to 30°C, 0.57 g of sodium periodate was added, and the mixture was stirred at 30°C for 2 hours. After reaction completion, the wet particles were recovered by filtration, and the recovered particles were washed 6 times with 200 mL of pure water to obtain the target formylated cellulose particles.

[Immobilization of Ligand to Formylated Cellulose Particles-1]

8.1 g of each ligand compound was dissolved in 11.2 g of pure water and heated to 30°C. Next, 15.0 g of the formylated cellulose particles obtained above were added, and the mixture was stirred at 30°C for 3 hours. Then, 0.06 g of sodium borohydride was added, and the mixture was stirred at 30°C for 2 hours. After reaction completion, the wet particles were recovered by filtration, and the recovered particles were washed 6 times with 50 mL of pure water to obtain the target products (carriers 10 and 11). The density of each ligand bound to the formylated cellulose particles is shown in Table 6.

[Table 6]

Table 6

| Carrier | Base carrier | Active group | Ligand compound | Ligand density (μmol/g) |
|---|---|---|---|---|
| Carrier 10 | Crosslinked cellulose particles A | Formyl group | 1,4-diaminobutane | 214 |
| Carrier 11 | Crosslinked cellulose particles A | Formyl group | 1,8-diaminooctane | 221 |

[Immobilization of Ligand to Formylated Cellulose Particles-2]

The ligand immobilization reaction was performed by the same method as above, except that the solvent for dissolving the ligand compound was changed to 8.9 g of methanol, and washing with 50 mL of methanol 3 times was added after reaction completion, to obtain the target products (carriers 12 and 13). The density of the ligand bound to the formylated cellulose particles is shown in Table 7.

[Table 7]

Table 7

| Carrier | Base carrier | Active group | Ligand compound | Ligand density (μmol/g) |
|---|---|---|---|---|
| Carrier 12 | Crosslinked cellulose particles A | Formyl group | 1,12-diaminododecane | 229 |
| Carrier 13 | Crosslinked cellulose particles A | Formyl group | 1,4-butanediol-bis(isopropylamine) | 207 |

(3) Vinyl Sulfonated Cellulose Particles

[Vinyl Sulfonation of Cellulose Particles]

1.4 g of sodium carbonate was dissolved in 25.9 g of pure water, and 16.1 g of the crosslinked cellulose particles A obtained in Example 1 was added to form a slurry. While stirring at 25°C, 1.5 g of divinyl sulfone was added, and the mixture was stirred at 25°C for 1 hour. After reaction completion, the wet particles were recovered by filtration, and the recovered particles were washed 6 times with 50 mL of pure water to obtain the target vinyl sulfonated cellulose particles.

[Immobilization of Ligand to Vinyl Sulfonated Cellulose Particles]

5.8 g of the ligand compound was dissolved in 6.9 g of methanol and heated to 30°C. Next, 10.0 g of the vinyl sulfonated cellulose particles obtained above was added, and the mixture was stirred at 30°C for 3 hours. After reaction completion, the wet particles were recovered by filtration, and the recovered particles were washed with 20 mL of methanol, then washed 6 times with 20 mL of pure water to obtain the target product (carrier 14). The density of the ligand bound to the vinyl sulfonated cellulose particles is shown in Table 8.

[Table 8]

Table 8

| Carrier | Base carrier | Active group | Ligand compound | Ligand density (μmol/g) |
|---|---|---|---|---|
| Carrier 14 | Crosslinked cellulose particles A | Vinyl sulfone group | 1,12-diaminododecane | 278 |

(4) Cellulose Particles B to F

Except for using the crosslinked cellulose particles B to F obtained in Example 1, carriers 15 to 20 and 24 were produced by the same method as the above carrier 12, carrier 21 was produced by the same method as the above carrier 10, and carrier 22 was produced by the same method as the above carrier 11, respectively. For carrier 19, in the formylation of cellulose particles, the addition amount of sodium periodate was changed to 0.23 g. The density of the ligand bound to the formylated cellulose particles is shown in Table 9.

[Table 9]

Table 9

| Carrier | Base carrier | Active group | Ligand | Ligand density (μmol/g) |
|---|---|---|---|---|
| Carrier 15 | Crosslinked cellulose particles B | Formyl group | 1,12-diaminododecane | 211 |
| Carrier 16 | Crosslinked cellulose particles C | Formyl group | 1,12-diaminododecane | 193 |
| Carrier 17 | Crosslinked cellulose particles D | Formyl group | 1,12-diaminododecane | 221 |
| Carrier 18 | Crosslinked cellulose particles E | Formyl group | 1,12-diaminododecane | 179 |
| Carrier 19 | Crosslinked cellulose particles E | Formyl group | 1,12-diaminododecane | 86 |
| Carrier 20 | Crosslinked cellulose particles E | Formyl group | 1,12-diaminododecane | 207 |
| Carrier 21 | Crosslinked cellulose particles E | Formyl group | 1,4-diaminobutane | 210 |
| Carrier 22 | Crosslinked cellulose particles E | Formyl group | 1,8-diaminooctane | 239 |
| Carrier 24 | Crosslinked cellulose particles F | Formyl group | 1,12-diaminododecane | 164 |

<Example 4: Antibody Purification Using Chromatography Carrier-1>

[Preparation of Antibody Solutions 1 to 9]

471 mL of CHO cell culture supernatant including monoclonal antibody (IgG1) was purified using protein A carrier Cellufine SPA-HC (manufactured by JNC Corporation) to obtain 50 mL of solution. Next, 0.1 M of hydrochloric acid was added to this solution until the pH reached 3.4, and the solution was allowed to stand at 25°C for 1 hour to perform virus inactivation. After virus inactivation, 1 M trishydroxyaminomethane aqueous solution was added to the solution until the pH reached 5.0. Since turbidity was confirmed, filtration was performed using a filter with a pore size of 0.45 μm. The concentration of monoclonal antibody in the filtrate was 19.6 mg/mL, and this was diluted with ultrapure water. Thereafter, using 1 M trishydroxyaminomethane aqueous solution and 5 M sodium chloride aqueous solution, the pH was adjusted to 7.0 and the electrical conductivity to 6 mS/cm to obtain an antibody solution.

The concentration of the antibody solution was calculated by dividing the absorbance at 280 nm by 1.40 using an absorbance meter. The HCP amount in the antibody solution was measured using an Elisa kit (Cygnus F-550-1). Using the obtained HCP amount and the monoclonal antibody amount calculated from the absorbance at a measurement wavelength of 280 nm, the HCP amount (ppm) was calculated from the formula {HCP amount in antibody solution (ng)/monoclonal antibody amount in antibody solution (mg)}. The concentration and HCP amount of the obtained antibody solution are shown in Table 10.

[Table 10]

Table 10

| **Antibody solution** | **Antibody concentration (mg/mL)** | **HCP (ppm)** |
|---|---|---|
| Antibody solution 1 | 10.5 | 1984 |
| Antibody solution 2 | 9.6 | 2421 |

(continued)

| Antibody solution | Antibody concentration (mg/mL) | HCP (ppm) |
|---|---|---|
| Antibody solution 3 | 10.0 | 2474 |
| Antibody solution 4 | 10.2 | 1718 |
| Antibody solution 5 | 9.9 | 1870 |
| Antibody solution 6 | 10.4 | 2045 |
| Antibody solution 7 | 10.1 | 3512 |
| Antibody solution 8 | 10.1 | 3165 |
| Antibody solution 9 | 10.5 | 2923 |

[Purification of Antibody Using Chromatography Carrier]

(Chromatography Carrier, Equipment, etc.)
Carrier: Carriers 2 to 8, 12, 14 to 17
Column: inner diameter 0.5 cm, height 3 cm
System: AKTA avant25
A buffer: 20 mM Tris-HCl buffer solution (pH 7.0)+NaCl (6 mS/cm)

(Procedure)

The carrier was packed into the column to a height of 1.5 cm. The column was connected to the system, and 20 column volumes of A buffer were passed through the column at 0.075 mL/min for equilibration. All subsequent processes were also performed at a flow rate of 0.075 mL/min. Next, the antibody solution was passed through the column to achieve an antibody loading amount of 200 g/L per unit volume of the chromatography carrier. Then, 10 column volumes of A buffer were passed through for washing. Subsequently, 5 column volumes of 0.1 M acetic acid aqueous solution were passed through. Next, 5 column volumes of 2 M sodium chloride solution were passed through. After that, 5 column volumes of 0.5 M sodium hydroxide aqueous solution were passed through for washing. Finally, 20 column volumes of A buffer were passed through for re-equilibration.

The entire 6 mL of column effluent during antibody solution passage and 3 mL of column effluent during non-adsorbed material washing were combined to form the recovery fraction. Table 11 shows the recovery rate of the monoclonal antibody (ratio of antibody recovery amount to antibody amount loaded onto the chromatography carrier) and the HCP amount in the recovery fraction. The recovery rate of the monoclonal antibody was calculated by the method described below.

(Recovery Rate of Monoclonal Antibody (%))

For both the antibody solution before purification and the obtained recovery fraction (antibody solution after purification), the absorbance at a measurement wavelength of 280 nm was measured using a spectrophotometer. The antibody concentration was calculated by dividing the absorbance at 280 nm by 1.40, and the recovery rate (%) was calculated using the formula (antibody amount in recovery fraction/antibody amount in antibody solution before purification)×100.

[Table 11]

Table 11

| Carrier | Antibody solution | Antibody recovery rate (%) | HCP amount (ppm) |
|---|---|---|---|
| Carrier 2 | Antibody solution 1 | 96 | 15 |
| Carrier 3 | Antibody solution 1 | 96 | 19 |

(continued)

| Carrier | Antibody solution | Antibody recovery rate (%) | HCP amount (ppm) |
|---|---|---|---|
| Carrier 4 | Antibody solution 1 | 95 | 18 |
| Carrier 5 | Antibody solution 1 | 98 | 15 |
| Carrier 6 | Antibody solution 1 | 95 | 7 |
| Carrier 7 | Antibody solution 1 | 96 | 18 |
| Carrier 8 | Antibody solution 2 | 94 | 18 |
| Carrier 12 | Antibody solution 2 | 93 | 3 |
| Carrier 14 | Antibody solution 2 | 95 | 8 |
| Carrier 15 | Antibody solution 3 | 95 | 3 |
| Carrier 16 | Antibody solution 3 | 93 | 9 |
| Carrier 17 | Antibody solution 3 | 95 | 4 |

From Table 11, it can be seen that by using the chromatography carrier according to the embodiment, a high antibody recovery rate was obtained and the amount of impurities (HCP) in the recovery fraction was small. Particularly with the chromatography carriers (carriers 6, 12, 14 to 17), the HCP amount was one order of magnitude lower.

<Example 5: Antibody Purification Using Chromatography Carrier-2>

Antibody purification was performed by the same method as Example 4, except that carriers 12 and 15 to 24 were used as chromatography carriers, antibody solutions 3, 4, and 7 to 9 were used as antibody solutions, and the antibody loading amount per unit volume of chromatography carrier was changed to 1010 g/L. Table 12 shows the recovery rate of the monoclonal antibody and the HCP amount in the recovery fraction.

In Table 12, chromatography carriers using Capto adhere as the carrier are comparative examples, and the antibodies were purified by the same method as above, except that Capto adhere (manufactured by Cytiva) was used. The ligand compound of Capto adhere is a quaternary amine having a benzyl group as a substituent.

[Table 12]

Table 12

| Carrier | Antibody solution | Antibody recovery rate (%) | HCP amount (ppm) |
|---|---|---|---|
| Carrier 12 | Antibody solution 3 | 97 | 297 |
| Carrier 15 | Antibody solution 3 | 98 | 133 |
| Carrier 16 | Antibody solution 3 | 100 | 99 |
| Carrier 17 | Antibody solution 3 | 100 | 23 |
| Carrier 18 | Antibody solution 4 | 99 | 8 |
| Carrier 19 | Antibody solution 7 | 99 | 87 |
| Carrier 20 | Antibody solution 7 | 97 | 39 |
| Carrier 21 | Antibody solution 8 | 98 | 53 |
| Carrier 22 | Antibody solution 8 | 98 | 14 |
| Carrier 23 | Antibody solution 9 | 96 | 18 |
| Carrier 24 | Antibody solution 4 | 99 | 7 |
| Capto adhere | Antibody solution 4 | 96 | 513 |

As can be seen from Table 12, even in the case of increasing the antibody loading amount to the column, using the

chromatography carrier according to the embodiment resulted in a high antibody recovery rate compared to the case of using Capto adhere, and the HCP amount in the recovery fraction was also significantly lower. A tendency was observed that the larger the average pore diameter of the porous particles, the lower the HCP amount in the recovery fraction, and particularly low HCP amounts were obtained in the case of using carriers 17, 18, 22, 23, and 24.

Although several embodiments of the present invention have been described, these embodiments are presented as examples and are not intended to limit the scope of the present invention. These novel embodiments can be implemented in various other forms, and various omissions, substitutions, and changes can be made without departing from the gist of the present invention. These embodiments and modifications thereof are included in the scope and gist of the present invention, and are included in the present invention described in the claims and the equivalent scope thereof.

## Claims

1. A chromatography carrier, comprising:

   a base carrier including porous particles; and a ligand immobilized on the base carrier,
   wherein the ligand is represented by the following formula (1), or is a polyallylamine having some amino groups modified or a salt thereof,

   $$-NH-Z-X \quad (1)$$

   (in formula (1), Z represents a divalent hydrophobic group; X represents $NH_2$ or represents a heterocyclic group including a nitrogen atom)
   the polyallylamine having some amino groups modified or a salt thereof comprises a structural unit represented by the following formula (2) and a structural unit represented by the following formula (3) or formula (3a), and has a weight average molecular weight of 1000 to 500000,

   [Chemical Formula 1]

   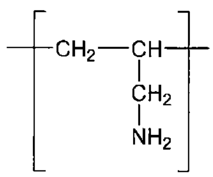
   (2)

   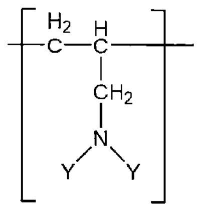
   (3)

   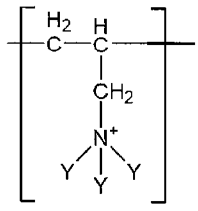
   (3a)

   [in formulas (3) and (3a), Y is each independently selected from the group consisting of a hydrogen atom, an alkyl group, an aryl group, $-COR^2$, and $-COOR^2$ (where $R^2$ is an alkyl group, an aryl group, an amino group, or an alkoxy group)]
   the ligand is immobilized on the base carrier via one or more types of active groups having reactivity to the ligand.

2. The chromatography carrier according to claim 1, wherein the ligand is represented by the formula (1).

3. The chromatography carrier according to claim 1 or 2, wherein Z in the formula (1) represents an alkylene group, a cycloalkylene group, -NR-, -O-, an arylene group, -S- or a combination thereof, each of which may have a substituent (where R represents a hydrogen atom, an alkyl group, an aryl group, an alkyl ester group, an aryl ester group, an alkyl ether group or an aryl ether group).

4. The chromatography carrier according to claim 3, wherein Z in the formula (1) represents an alkylene group having 2 to 12 carbon atoms, a cycloalkylene group having 3 to 6 carbon atoms, -O-, a phenylene group or a combination thereof.

5. The chromatography carrier according to claim 1, wherein the ligand is a polyallylamine having some amino groups modified, which comprises a structural unit represented by the formula (2) and a structural unit represented by the formula (3), and in the formula (3), one of Y is an alkyl group having 1 to 12 carbon atoms, a phenyl group, or $-COR^2$ (where $R^2$ is an alkyl group having 1 to 12 carbon atoms or a phenyl group), and the other is a hydrogen atom.

6. The chromatography carrier according to claim 1, wherein the ligand comprises a group derived from one or more compounds selected from the group consisting of 1,2-diaminoethane, 1,3-diaminopropane, 1,4-diaminobutane, 2-methyl-1,3-propanediamine, 1,5-diaminopentane, 1,3-diaminopentane, 1,6-diaminohexane, 3,3'-diaminodipropylamine, 3,3'-diamino-N-methyldipropylamine, 2-methyl-1,5-diaminopentane, 1,4-diaminocyclohexane, 1,7-diaminoheptane, 1,8-diaminooctane, 4-(2-aminoethyl)cyclohexylamine, 1,9-diaminononane, 1,10-diaminodecane, 1,4-butanediol-bis(isopropylamine), 1,11-diaminoundecane, 1,12-diaminododecane, N,N-bis(3-aminopropyl)1,4-butanediamine, 6,6'-diaminodihexylamine, p-xylylenediamine, 4-picolylamine, and a polyallylamine having some amino groups modified, which comprises a structural unit represented by the following formula (2') and a structural unit represented by the following formula (3'),

[Chemical Formula 2]

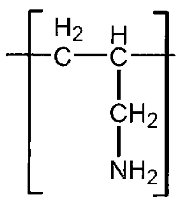
(2')

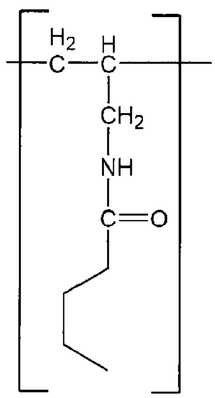
(3')

7. The chromatography carrier according to any one of claims 1 to 6, wherein the active groups are selected from the group consisting of an epoxy group, a formyl group, and a vinyl sulfone group.

8. The chromatography carrier according to any one of claims 1 to 7, wherein the porous particles are crosslinked cellulose.

9. The chromatography carrier according to any one of claims 1 to 8, wherein the porous particles have an average pore diameter of 200 nm or more.

10. A method for purifying an antibody, comprising: a step of bringing a solution including an antibody and a host cell protein (HCP) into contact with the chromatography carrier according to any one of claims 1 to 9 to obtain an antibody solution with a reduced HCP content.

11. The method according to claim 10, wherein the step of bringing the solution including the antibody and the host cell protein (HCP) into contact with the chromatography carrier is performed by passing the solution through a container containing the chromatography carrier in a flow-through mode.

12. The method according to claim 10 or 11, wherein the antibody is a monoclonal antibody.

13. The method according to any one of claims 10 to 12, wherein the HCP content in the antibody solution is 300 ppm or less.

**INTERNATIONAL SEARCH REPORT**

International application No.
**PCT/JP2024/030807**

**A. CLASSIFICATION OF SUBJECT MATTER**

***B01J 20/281***(2006.01)i; ***B01D 15/38***(2006.01)i; ***B01J 20/28***(2006.01)i; ***B01J 20/285***(2006.01)i; ***C07K 1/16***(2006.01)i; ***C07K 16/00***(2006.01)i; ***G01N 30/88***(2006.01)i
FI: B01J20/281 R; B01D15/38; B01J20/22 D; B01J20/28 Z; B01J20/281 G; B01J20/281 X; B01J20/285 T; C07K1/16; C07K16/00; G01N30/88 J

According to International Patent Classification (IPC) or to both national classification and IPC

**B. FIELDS SEARCHED**

Minimum documentation searched (classification system followed by classification symbols)

B01J20/281; B01D15/38; B01J20/28; B01J20/285; C07K1/16; C07K16/00; G01N30/88

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | WO 2018/092691 A1 (JNC CORPORATION) 24 May 2018 (2018-05-24) claims, paragraphs [0049]-[0051], [0074]-[0134] | 1, 5-13 |
| Y | | 8, 10-13 |
| X | JOHANSSON, Bo-Lennart et al., Journal of Chromatography A, 2003, vol. 1016, pp. 21-33 abstract, page 22, right column, table 1 | 1-4, 6-7, 9 |
| Y | | 8, 10-13 |
| X | LI, Ming et al., Biochemical Engineering Journal, 2019, vol. 142, pp. 153-161 abstract, page 154, right column | 1, 5, 7, 9 |
| Y | | 8, 10-13 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 September 2024** | **08 October 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No. **PCT/JP2024/030807**

**C. DOCUMENTS CONSIDERED TO BE RELEVANT**

| Category* | Citation of document, with indication, where appropriate, of the relevant passages | Relevant to claim No. |
|---|---|---|
| X | FARZI-KHAJEH, Hamed et al., Journal of Chromatography A, 2022, vol. 1684, no. 463559, pp. 1-11<br>abstract, pages 2-3 | 1-3, 7, 9 |
| Y | | 8, 10-13 |
| X | WO 2014/069474 A1 (KURARAY CO., LTD.) 08 May 2014 (2014-05-08)<br>claims, examples | 1-4, 6, 9 |
| X | MAJER, Janja et al., Macromol. Symp., 2019, vol. 296, pp. 5-10<br>scheme 1, table 1, conclusion | 1-4, 6-7 |

Form PCT/ISA/210 (second sheet) (July 2022)

**INTERNATIONAL SEARCH REPORT**
**Information on patent family members**

International application No.
**PCT/JP2024/030807**

| Patent document cited in search report | Publication date (day/month/year) | Patent family member(s) | Publication date (day/month/year) |
|---|---|---|---|
| WO 2018/092691 A1 | 24 May 2018 | US 2019/0345194 A1<br>claims, paragraphs [0097]-[0099], [0124]-[0296]<br>CN 109964123 A | |
| WO 2014/069474 A1 | 08 May 2014 | US 2015/0224472 A1<br>claims, examples<br>EP 2915840 A1<br>CN 104755543 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 5064225 B
- JP 2018092691 A
- JP 2021046284 A
- JP 2009242770 A
- JP 55039565 A
- JP 55040618 A
- JP 2023037724 A
- JP 63062252 A
- JP 59038203 A
- JP 3663666 B

### Non-patent literature cited in the description

- *Journal of Chromatography A*, 2003, vol. 1016, 21-33